# EUROPEAN PATENT SPECIFICATION

(11) **EP 2 252 365 B1**
(45) Date of publication and mention of the grant of the patent: **16.10.2013**
(21) Application number: 09712317.8
(22) Date of filing: 20.02.2009
(51) Int. Cl.: A61N 1/08, A61N 1/372, H01B 7/36

(54) **TEMPORARY NEUROSTIMULATION LEAD IDENTIFICATION DEVICE**
TEMPORÄRE IDENTIFIZIERUNGSVORRICHTUNG FÜR NEUROSTIMULATIONSLEITER
DISPOSITIF TEMPORAIRE D'IDENTIFICATION D'UN FIL DE NEUROSTIMULATION

(30) Priority: 21.02.2008 US 30506 P
(43) Date of publication of application: 24.11.2010
(73) Proprietor: Boston Scientific Neuromodulation Corporation, Valencia, CA 91355 (US)
(72) Inventor: BARKER, John M., Ventura California 93003 (US)
(74) Representative: Vossius & Partner
(86) International application number: PCT/US2009/034683
(87) International publication number: WO 2009/105646

(56) References cited:
- WO-A-03/008037
- WO-A-03/020365
- WO-A-03/092794
- US-A- 4 628 934
- US-A1- 2002 147 485
- US-A1- 2006 020 314

## Description

### FIELD OF THE INVENTION

The present invention relates to tissue stimulation systems, and more particularly, to apparatus and methods for identifying neurostimulation leads.

### BACKGROUND OF THE INVENTION

Implantable neurostimulation systems have proven therapeutic in a wide variety of diseases and disorders. Pacemakers and Implantable Cardiac Defibrillators (ICDs) have proven highly effective in the treatment of a number of cardiac conditions (e.g., arrhythmias). Spinal Cord Stimulation (SCS) systems have long been accepted as a therapeutic modality for the treatment of chronic pain syndromes, and the application of tissue stimulation has begun to expand to additional applications such as angina pectoralis and incontinence. Deep Brain Stimulation (DBS) has also been applied therapeutically for well over a decade for the treatment of refractory chronic pain syndromes, and DBS has also recently been applied in additional areas such as movement disorders and epilepsy. Further, in recent investigations Peripheral Nerve Stimulation (PNS) systems have demonstrated efficacy in the treatment of chronic pain syndromes and incontinence, and a number of additional applications are currently under investigation. Also, Functional Electrical Stimulation (FES) systems such as the Freehand system by NeuroControl (Cleveland, Ohio) have been applied to restore some functionality to paralyzed extremities in spinal cord injury patients.

Each of these implantable neurostimulation systems typically includes one or more stimulation leads implanted at the desired stimulation site and an implantable neurostimulator, such as an implantable pulse generator (IPG), implanted remotely from the stimulation site, but coupled either directly to the stimulation leads or indirectly to the stimulation leads via one or more extension leads in cases where the length of the stimulation leads is insufficient to reach the IPG. In some cases, the extension leads may be used to facilitate coupling of the neurostimulator, which may otherwise be incompatible with the stimulation leads or extension leads, thereto. Thus, electrical pulses can be delivered from the neurostimulator to the stimulation leads to stimulate the tissue and provide the desired efficacious therapy to the patient.

If the stimulation leads are to be directly connected to the neurostimulator, the proximal ends of the stimulation leads can be inserted into a connector of the neurostimulator, such that the terminals located at the proximal ends of the stimulation leads are coupled to corresponding electrical contacts within the connector. Individual wires are routed through each stimulation lead to connect the proximally-located terminals with the distally-located electrodes.

If the stimulation leads are to be indirectly connected to the neurostimulator via the extension leads, the proximal ends of the stimulation leads can be inserted into connectors located at the distal ends of the respective extension leads, such that the terminals of the stimulation leads are coupled to corresponding electrical contacts within the connectors of the extension leads. The proximal ends of the extension leads can then be inserted into the connector of the neurostimulator, such that terminals located at the proximal ends of the extension leads are coupled to the corresponding electrical contacts within the connector of the neurostimulator. Individual wires are routed through each extension lead to respectively couple the proximally-located terminals to the distally-located electrical contacts.

In the context of an SCS procedure, one or more stimulation leads are introduced through the patient's back into the epidural space under fluoroscopy, such that the electrodes carried by the leads are arranged in a desired pattern and spacing to create an electrode array. The specific procedure used to implant the stimulation leads will ultimately depend on the type of stimulation leads used. Currently, there are two types of commercially available stimulation leads: a percutaneous lead and a surgical lead.

A percutaneous lead comprises a cylindrical body with ring electrodes, and can be introduced into contact with the affected spinal tissue through a Touhy-like needle, which passes through the skin, between the desired vertebrae, and into the epidural space above the dura layer. For unilateral pain, a percutaneous lead is placed on the corresponding lateral side of the spinal cord. For bilateral pain, a percutaneous lead is placed down the midline of the spinal cord, or two percutaneous leads are placed down the respective sides of the midline. In many cases, a stylet, such as a metallic wire, is inserted into a lumen running through the center of each of the percutaneous leads to aid in insertion of the lead through the needle and into the epidural space. The stylet gives the lead rigidity during positioning, and once the lead is positioned, the stylet can be removed after which the lead becomes flaccid.

A surgical lead has a paddle on which multiple electrodes are arranged in independent columns, and is introduced into contact with the affected spinal tissue using a surgical procedure, and specifically, a laminectomy, which involves removal of the laminar vertebral tissue to allow both access to the dura layer and positioning of the lead.

After proper placement of the stimulation leads at the target area of the spinal cord, the leads are anchored in place at an exit site to prevent movement of the stimulation leads. To facilitate the location of the neurostimulator away from the exit point of the stimulation leads, extension leads are sometimes used. In particular, the proximal ends of the stimulation leads, which include terminals respectively coupled to the electrodes on the stimulation leads, are inserted into connectors located at the distal ends of extension leads. Whether extension leads are used or not, the proximal ends of the stimulation leads exiting the spinal column are passed through a tunnel subcutaneously formed along the torso of the patient to a subcutaneous pocket (typically made in the patient's abdominal or buttock area) where a neurostimulator is implanted. The subcutaneous tunnel can be formed using a tunneling tool over which a tunneling straw may be threaded. The tunneling tool can be removed, the stimulation leads threaded through the tunneling straw, and then the tunneling straw removed from the tunnel while maintaining the stimulation leads in place within the tunnel.

The stimulation leads are then connected directly to the neurostimulator by inserting the proximal ends of the stimulation leads within one or more connector ports of the IPG or connected to extension leads, which are then inserted into the connector ports of the IPG. The IPG can then be operated to generate electrical pulses that are delivered, through the electrodes, to the targeted tissue, and in particular, the dorsal column and dorsal root fibers within the spinal cord. The stimulation creates the sensation known as paresthesia, which can be characterized as an alternative sensation that replaces the pain signals sensed by the patient. During the surgical procedure, the neurostimulator may be operated to test the effect of stimulation and adjust the parameters of the stimulation for optimal pain relief. The patient may provide verbal feedback regarding the presence of paresthesia over the pain area, and based on this feedback, the lead positions may be adjusted and re-anchored if necessary. Any incisions are then closed to fully implant the system.

Oftentimes, multiple leads may extend from the spinal region of the patient. For example, multiple stimulation leads may be implanted within the patient adjacent the spinal cord, or in the case of paddle leads, multiple lead tails may extend from the paddle, with each lead tail being coupled to specific electrodes on the paddle. Because the programming of the IPG will depend upon the physical locations of the electrodes relative to the patient's spinal cord, the proximal ends of the leads are labeled before passing them through the tunneling straw, so that the surgeon can keep track of which set of electrodes is connected to which connector port on the implanted IPG (which may include up to four ports in the near future), or if multiple IPGs are to be implanted, which set of electrodes is connected to which IPG.

One technique used by surgeons to identify the leads is to tie sutures around the proximal ends of the leads prior to introducing them through the tunneling straw; for example, one suture around a first lead, two sutures around a second lead, three sutures around a third lead, etc. Once the proximal ends of the leads exit the tunneling straw, the surgeon can then identify each lead by the number of sutures tied to the respective lead, thereby allowing the lead to be connected to the correct port on the IPG.

While this technique can be successfully employed to identify leads, it considerably extends the length of the surgery time, which is undesirable. In some cases, the identification features, such as different colors or markings, can be incorporated into the proximal ends of the leads, such that the leads can be identified as they exit the tunneling straw. However, this requires the surgeon to maintain a collection of leads with several different identification marks, which cannot easily be accomplished given the fact that different procedures require different numbers of leads, thereby requiring the surgeon to mix and match leads for each procedure.

There, thus, remains a need for a quick, effective, and low-cost method for temporarily identifying a lead that would not require changes in surgical techniques or existing surgical tools (e.g., insertion needles, tunneling straws, etc.).

Published patent application WO03/092794 A2 discloses an electrical lead having an elongated lead body, an electrode and a proximal-end lumen. It further discloses a temporary identification device including a handle, a shaft and an identifier, said shaft being sized to be firmly and removably received within said lumen of the electrical lead. In this document, said temporary identification device is used to identify a port of an implantable device adapted to receive a particular type of lead.

### SUMMARY OF THE INVENTION

In accordance with the present invention, an implantable lead kit comprises an electrical lead including an elongated lead body having a proximal end, at least one distally-located electrode, and a lumen disposed within the proximal end of the lead body. In one embodiment, the electrical lead is a percutaneous lead, in which case, the lead body has a distal end, and each of the electrode(s) is a ring-electrode mounted around a circumference of the distal end of the lead body. In another embodiment, the electrical lead is a surgical lead that includes a paddle-shaped membrane from which the lead body extends, and each of the electrode(s) is mounted on one side of the paddle-shaped membrane. The electrical lead may further include at least one terminal carried by the proximal end of the lead body and at least one electrical conductor respectively connected between the electrode(s) and the terminal(s).

The implantable lead kit further comprises a plurality of temporary identification devices, each of which includes a handle having a different identifier and a shaft extending from the handle. Each shaft is sized to be firmly and removably received within the lumen of the electrical lead. In one embodiment, if the first electrical lead is a percutaneous lead, the implantable lead kit may further comprise another percutaneous lead including an elongated body having a proximal end, at least one distally-located electrode, and a lumen disposed within the proximal end of the elongated body, wherein each shaft is sized to be firmly and removably received within the lumen of the other percutaneous lead. In another embodiment, if the electrical lead is a surgical lead, it may further include another elongated lead body having a proximal end, and another lumen disposed within the proximal end of the other lead body, wherein each shaft is sized to be firmly and removably received within other lumen of the surgical lead.

In one embodiment, each handle has a distal-facing surface sized to abut a proximal-facing surface of the lead body when the respective shaft is fully received within the lumen of the electrical lead. In another embodiment, each handle has a distal end having substantially the same cross-sectional size as a cross-sectional size of the proximal end of the lead body. In still another embodiment, each handle is tapered downward in the proximal direction. The different identifier can take the form of any feature that can be used to differentiate the lead bodies. For example, the different identifier may be an alpha-numeric character, a color, and/or a geometric shape.

The implantable lead kit may further comprise a stylet sized to be removably received within the lumen of the electrical lead, a tunneling tool configured for subcutaneously creating a tunnel within a patient, and a tunneling straw configured for being introduced within the tunnel and further configured for receiving the proximal end of the lead body.

Other and further aspects and features of the invention will be evident from reading the following detailed description of the preferred embodiments, which are intended to illustrate, not limit, the invention.

### BRIEF DESCRIPTION OF THE DRAWINGS

The drawings illustrate the design and utility of preferred embodiments of the present invention, in which similar elements are referred to by common reference numerals. In order to better appreciate how the above-recited and other advantages and objects of the present inventions are obtained, a more particular description of the present inventions briefly described above will be rendered by reference to specific embodiments thereof, which are illustrated in the accompanying drawings. Understanding that these drawings depict only typical embodiments of the invention and are not therefore to be considered limiting of its scope, the invention will be described and explained with additional specificity and detail through the use of the accompanying drawings in which:
**Fig. 1** is a plan view of one embodiment of a prior art tissue stimulation system;
**Fig. 2** is a cross-sectional view of a stimulation lead used in the tissue stimulation system of **Fig. 1****,** taken along the line **2-2**;
**Fig. 3** is a plan view of another embodiment of a prior art tissue stimulation system;
**Fig. 4** is a plan view of the tissue stimulation system of **Fig. 1** in use with a patient;
**Fig. 5** is a perspective view of one embodiment of a lead assembly kit arranged in accordance with present invention;
**Fig. 6** is a perspective of view of temporary identification devices that are used in the lead assembly kit of **Fig. 5**;
**Fig. 7** is a close-up perspective view of one of the temporary identification devices of **Fig. 6**;
**Fig. 8** is another close-up perspective view of one of the temporary identification devices of **Fig. 6**;
**Fig. 9** is a cross-sectional view of the temporary identification device of **Fig. 7**, taken along the axis of the device;
**Fig. 10** is a cross-sectional view of the temporary identification device of Fig. 7, taken transversely to the axis of the device;
**Fig. 11** is a close-up perspective view of the temporary identification device of **Fig. 6** mounted to the proximal end of the lead body used in the lead assembly kit of **Fig. 5**; and
**Figs. 12-16** are plan views of a method for implanting a plurality of stimulation leads into a patient using the lead assembly kit of **Fig. 5****.**

### DETAILED DESCRIPTION OF THE EMBODIMENTS

The description that follows relates to a spinal cord stimulation (SCS) system. However, it is to be understood that while the invention lends itself well to applications in SCS, the invention, in its broadest aspects, may not be so limited. Rather, the invention may be used with any type of implantable electrical circuitry used to stimulate tissue. For example, the present invention may be used as part of a pacemaker, a defibrillator, a cochlear stimulator, a retinal stimulator, a stimulator configured to produce coordinated limb movement, a cortical stimulator, a deep brain stimulator, peripheral nerve stimulator, microstimulator, or in any other neural stimulator configured to treat urinary incontinence, sleep apnea, shoulder sublaxation, headache, etc.

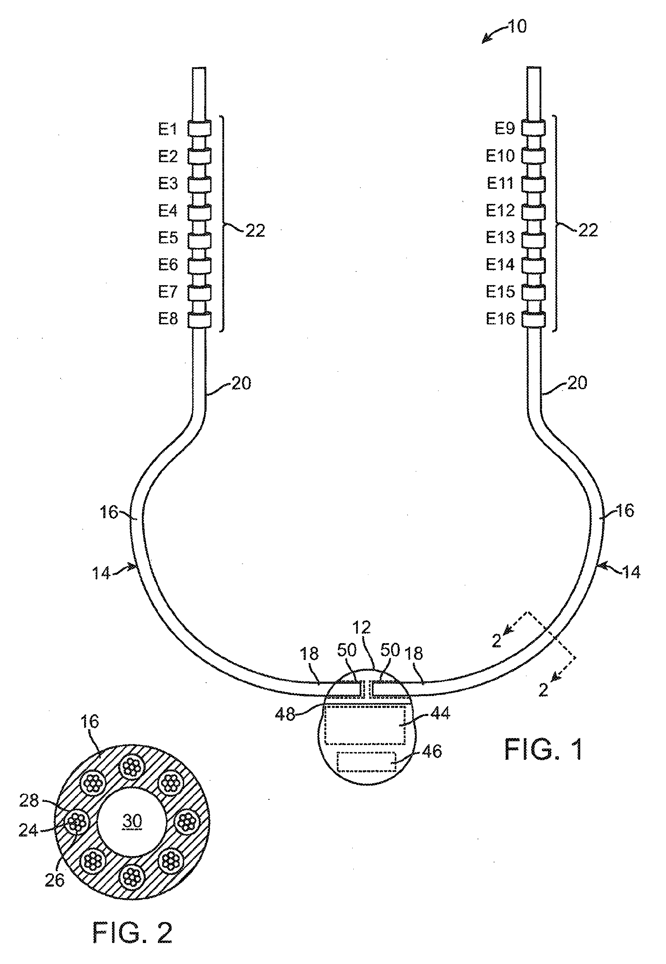

Referring first to **Fig. 1**, a generalized tissue stimulation system 10 that may be used in spinal cord stimulation (SCS), as well as other stimulation applications, will be described. The stimulation system 10 generally comprises an implantable neurostimulator 12, and a plurality of implantable stimulation leads 14 (in this case, two leads) coupled to the neurostimulator. The system 10 may optionally comprise a plurality of extension leads (not shown) through which the stimulation leads 14 can be indirectly coupled to the neurostimulator 12.

In the embodiment illustrated in **Fig. 1**, each stimulation lead 14 takes the form of a percutaneous lead that comprises an elongated lead body 16 having a proximal end 18 and a distal end 20. The lead body 16 may, e.g., have a diameter within the range of 0.76 to 1.78 mm (0.03 inches to 0.07 inches) and a length within the range of 30cm to 90cm for spinal cord stimulation applications. The lead body 16 may be composed of a suitable electrically insulative material, such as, a polymer (e.g., polyurethane or silicone), and may, be extruded as a unibody construction.

Each stimulation lead 14 further comprises a plurality of terminals (not shown) mounted to the proximal end 18 of the lead body 16 and a plurality of in-line electrodes 22 (in this case, electrodes E1-E8 for the first lead and electrodes E9-E16 for the second lead) mounted to the distal end 20 of the lead body 16. The electrodes 22 are shown exaggerated for purposes of illustration. Although the stimulation lead 14 is shown as having eight electrodes 22 (and thus, eight corresponding terminals), the number of electrodes may be any number suitable for the application in which the stimulation lead 14 is intended to be use (e.g., two, four, sixteen, etc.). Each of the electrodes 22 takes the form of a cylindrical ring element composed of an electrically conductive, non-corrosive, material, such as, e.g., platinum, titanium, stainless steel, or alloys thereof, which is circumferentially disposed about the lead body 16.

As shown in **Fig. 2**, the stimulation lead 14 also includes a plurality of electrical conductors 24 (each comprising individual strands 26) extending through individual lumens 28 within the lead body 16 and connected between the respective terminals (not shown) and electrodes 22 using suitable means, such as welding, thereby electrically coupling the proximally-located terminals with the distally-located electrodes 22. The stimulation lead 14 further includes a central lumen 30 that may be used to accept an insertion stylet (described in further detail below) to facilitate lead implantation. As will also be described in further detail below, the central lumen 30 is also capable of receiving a temporary identification device to allow the stimulation lead 14 to be more easily identified, so that the lead body 16 associated with a specific set of electrodes 22 (either electrodes E1-E8 or electrodes E9-E16) can be more easily determined. The central lumen 30 may, e.g., have a diameter within the range of about 0.25 - 0.76 mm (.01-.03 inches).

Further details describing the construction and method of manufacturing stimulation leads are disclosed in U.S. Patent Publication No. 2007-0168007and U.S. Patent Publication No. 2007-0168004.

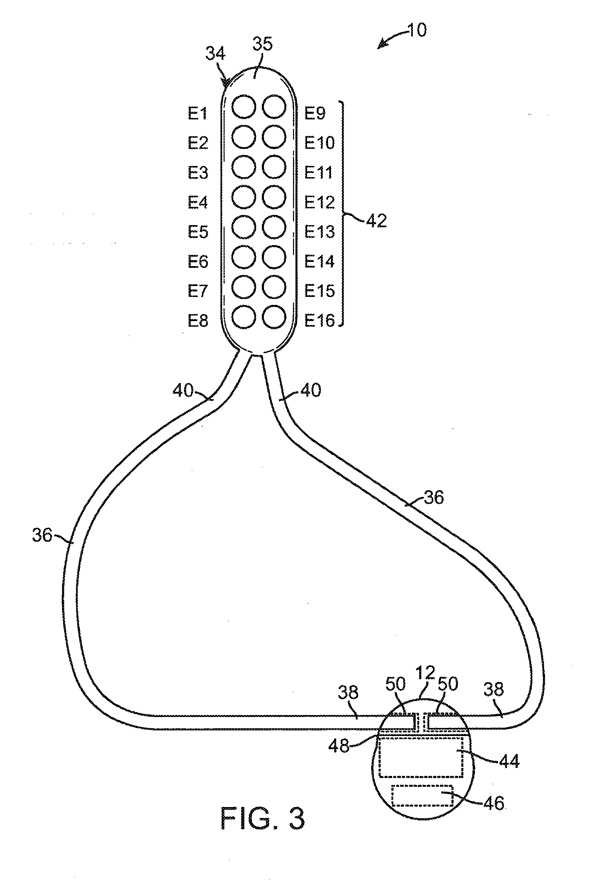

Alternatively, rather than percutaneous leads, a single surgical stimulation lead 34 may be used, as shown in **Fig. 3**. The surgical lead 34 comprises a paddle-shaped membrane 35, and two elongated lead bodies 36 extending from the paddle-shaped membrane 36. Each of the lead bodies 36 has a proximal end 38 and distal end 40. Each lead body 36 may, e.g., have a diameter within the range 0.76 to 1.78 mm (0.03 inches to 0.07 inches) and a length within the range of 30cm to 90cm for spinal cord stimulation applications. Each lead body 36 may be composed of a suitable electrically insulative material, such as, a polymer (e.g., polyurethane or silicone), and may be extruded as a unibody construction. The paddle-shaped membrane 35 is composed of an electrically insulative material, such as silicone.

The stimulation lead 34 further comprises a plurality of terminals (not shown) mounted to the proximal end 38 of each lead body 36 and a plurality of electrodes 42 mounted on one side of the paddle-shaped membrane 35 in a two-dimensional arrangement (in this case, two columns of electrodes, and in this case, electrodes E1-E8 for the first column, and electrodes E9-E16 for the second column). Although the stimulation lead 34 is shown as having sixteen electrodes 42 (and thus, sixteen corresponding terminals on each lead body 36), the number of electrodes may be any number suitable for the application in which the stimulation lead 34 is intended to be use (e.g., two, four, eight, etc.). Each of the electrodes 42 takes the form of a disk composed of an electrically conductive, non-corrosive, material, such as, e.g., platinum, titanium, stainless steel, or alloys thereof.

In the same manner described above with respect to the stimulation lead 14 shown in **Fig. 2**, the stimulation lead 34 also includes a plurality of electrical conductors (not shown) extending through individual lumens (not shown) within each lead body 36 and connected between the respective terminals (not shown) and electrodes 42 using suitable means, such as welding, thereby electrically coupling the proximally-located terminals on each lead body 36 to a specific column of electrodes 42 located on the paddle-shaped membrane 35 (in this case, one lead body is coupled to electrodes E1-E8, and the other lead body is coupled to electrodes E9-E16). Each lead body 36 of the stimulation lead 34 includes a central lumen (not shown) capable of receiving a temporary identification device to allow each lead body 16 to be more identified, so that the lead body 16 associated with a specific column of electrodes 22 (either electrodes E1-E8 or electrodes E9-E16) can be more easily determined. The central lumen may, e.g., have a diameter within the range of about 0.25 to 0.76 mm (0.01 inches to 0.03 inches).

Referring to either of **Figs. 1** or **3**, the neurostimulator 12 takes the form of an implantable pulse generator (IPG) that comprises an electronic subassembly 44 (shown in phantom), which includes control and pulse generation circuitry (not shown) for delivering electrical stimulation energy to the electrodes (described below) of the stimulation lead 14 in a controlled manner, and a power supply, e.g., a battery 46 (shown in phantom), so that once programmed and turned on by an external programming device (not shown), the neurostimulator 12 can operate independently of external hardware.

Alternatively, the neurostimulator 12 can take the form of an implantable receiver-stimulator (not shown), in which case, the power source, e.g., a battery, for powering the implanted receiver, as well as control circuitry to command the receiver-stimulator, will be contained in an external controller inductively coupled to the receiver-stimulator via an electromagnetic link. Alternatively, the neurostimulator 12 can take the form of an external trial stimulator (ETS)(not shown), which has similar pulse generation circuitry as an IPG, but differs in that it is a non-implantable device that is used on a trial basis after the stimulation lead 14 has been implanted and prior to implantation of the IPG, to test the responsiveness of the stimulation that is to be provided.

The neurostimulator 12 comprises an outer housing 48 for housing the electronic and other components (described in further detail below), and connectors 50 to which the proximal ends of the respective lead bodies 16 (**Fig. 1**) or lead bodies 36 (**Fig. 3**) mates in a manner that electrically couples the electrodes 22 (**Fig. 1**) or 42 (**Fig. 3**) to the pulse generation circuitry contained within the outer housing 48. While the ports for the connectors 50 are shown in **Figs. 1** and **3** as being on opposite sides of each other, the ports may be on the same side. The outer housing 48 is composed of a biocompatible material, such as titanium, and forms a hermetically sealed compartment wherein the electronic subassembly 44 and battery 46 are protected from the body tissue and fluids. The connectors 50 are disposed in a portion of the housing 48 that is, at least initially, not sealed.

Each of the connectors 50 carries a plurality of contacts (not shown) that come into electrical contact with the respective terminals of the respective stimulation lead 14 (**Fig. 1**) or stimulation lead 34 (**Fig. 3**) when the proximal end 18 of the respective lead body 16 (**Fig. 1**) or proximal end 38 of the respective lead body 36 (**Fig. 3**) is inserted into the connector 50. Electrical conductors (not shown), which extend from each connector 50 in electrical contact with the contacts, penetrate the housing 48 into the sealed chamber and connect to the electronic subassembly 44. Additional details discussing neurostimulators, including the outer housing 48 and connectors 50, are disclosed in U.S. Patent Publication No. 2007-0161294.

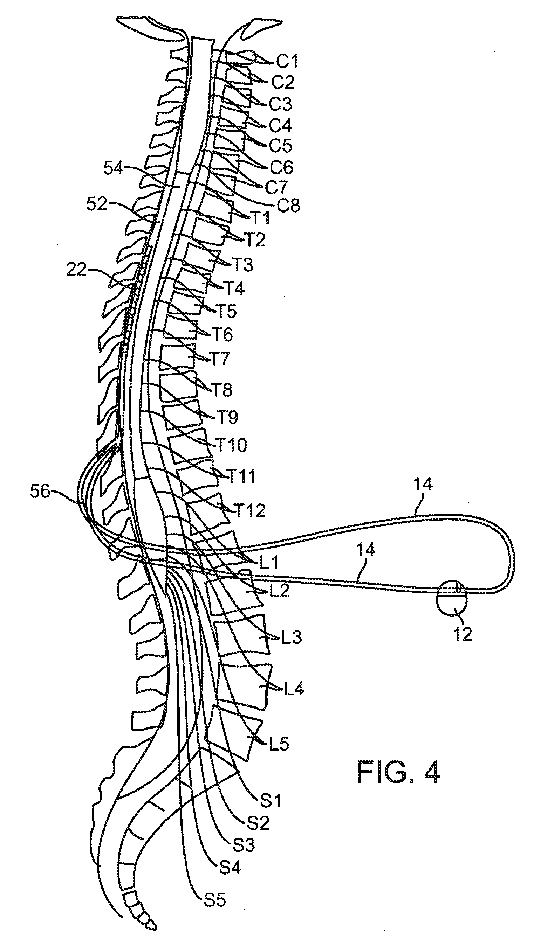

As shown in **Fig. 4**, the stimulation leads 14 (or alternatively the stimulation lead 34) are implanted in the epidural space 52 of a patient in close proximity to the spinal cord 54. The preferred placement of the stimulation leads 14 is such that the electrode array 22 is adjacent (i.e., resting upon) the dura nearest the target area of the spinal cord 54. Because of the lack of space near the lead exit point 56 where the stimulation leads 14 exit the spinal column, the neurostimulator 12 is generally implanted in a surgically-made pocket either in the abdomen or above the buttocks. The neurostimulator 12 may, of course, also be implanted in other locations of the patient's body. If necessary, the optional extension leads (not shown) may facilitate locating the neurostimulator 12 away from the lead exit point 56.

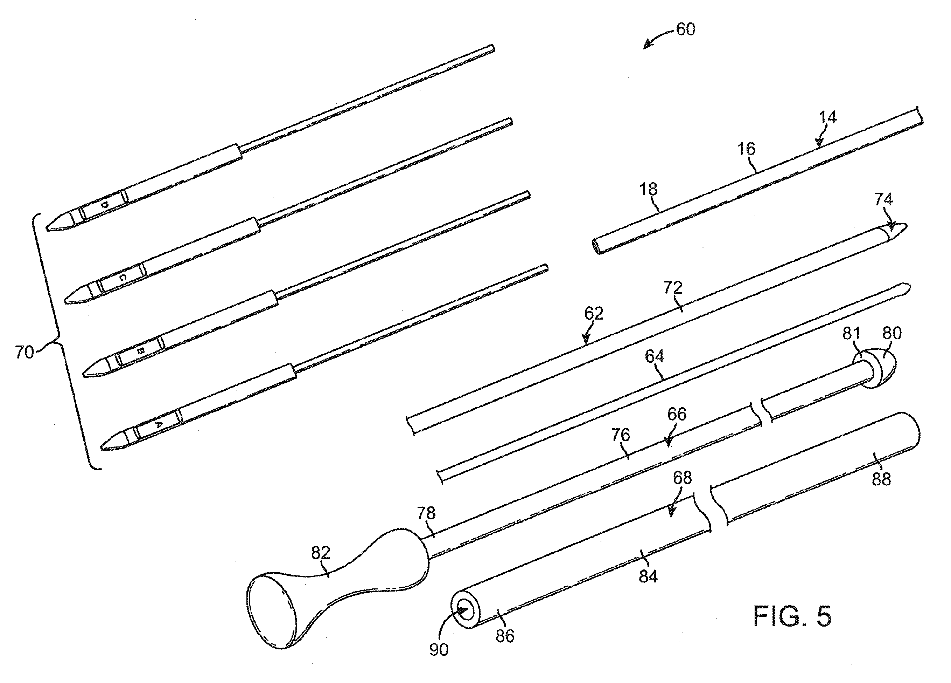

Referring further to **Fig. 5**, a lead assembly kit 60 arranged in accordance with one embodiment of the present inventions will now be described. The lead assembly kit 60 comprises the previously described percutaneous stimulation leads 14 (only one shown in **Fig. 5**) or alternatively the stimulation lead 34, a hollow needle 62, a stylet 64, a tunneling tool 66, a tube, and in particular a tunneling straw 68, and a plurality of temporary identifying devices 70. With the exception of the temporary identifying devices 70, the components of the kit 60 are conventional.

The hollow needle 62 is a standard epidural needle that includes an elongated needle shaft 72 and a lumen 74 extending through the needle shaft 72, and the stylet 64 is composed of a semi-rigid shaft that is sized to be disposed within the lumen 74 of the needle 62. The tunneling tool 66 includes an elongated semi-rigid shaft 76 having a proximal end 78 and an atraumatic blunt distal tip 80, and a handle 82 removably mounted to the proximal end 78 of the rigid shaft 76, e.g., using a threaded arrangement. The tunneling straw 68 comprises an elongated hollow cylindrical body 84 having a proximal end 86 and a distal end 88, and a lumen 90 extending through the cylindrical body 84. The lumen 90 of the tunneling straw 68 is sized to separately receive the shaft 76 of the tunneling tool 66 and the combination of lead bodies 16 of the stimulation leads 14 (or alternatively, the combination of lead bodies 36 of the stimulation lead 34). As will be described in further detail below, during a tunneling procedure, the tunneling straw 68 fits over the shaft 76 of the tunneling tool 66 between a flange 81 of the blunt distal tip 80 and a distal-facing surface of the handle 82. Thus, the outer diameter of the tip 80 is preferably the same as the outer diameter of the tunneling straw 68 to provide the assembly with a continuous exterior surface.

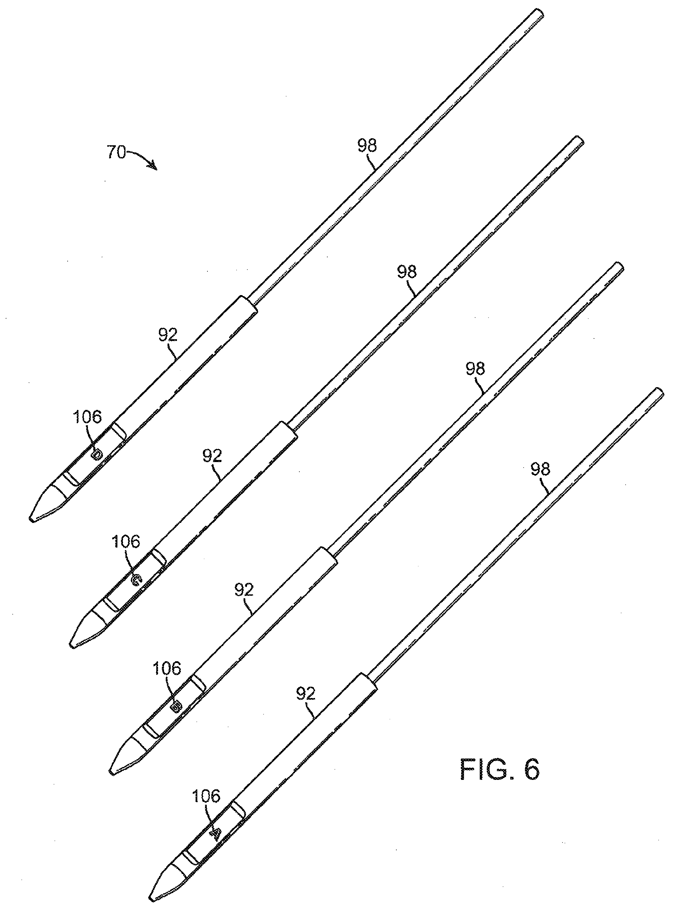

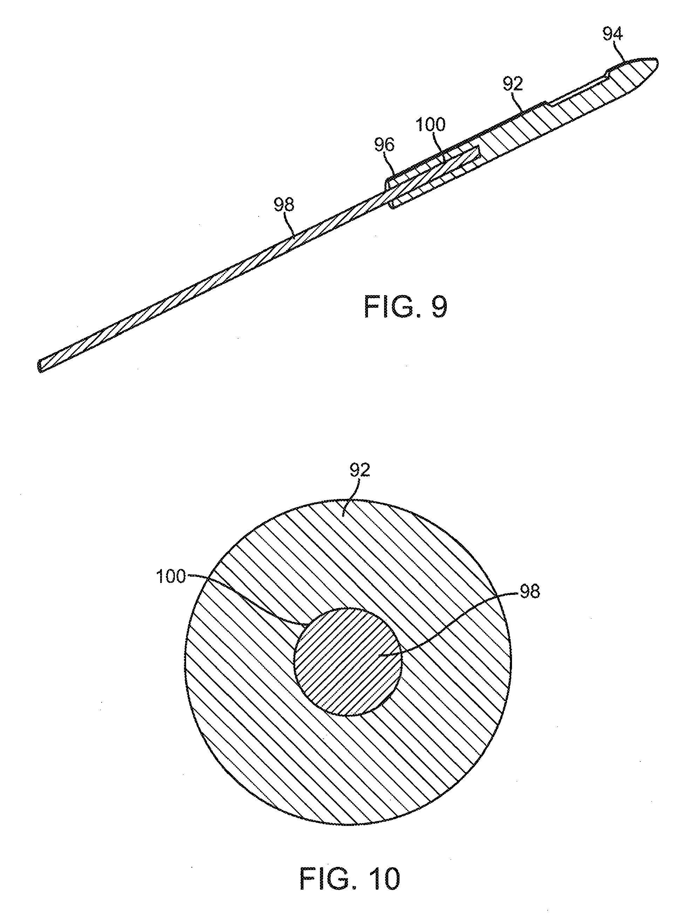

In the illustrated embodiment, four identification devices 70 are shown, although any plural number of identification devices can be used (e.g., 2, 3, 5, 6, 7, 8, etc.). Referring further to **Figs. 6-10**, each identification device 70 generally comprises a handle 92 having a proximal end 94 and a distal end 96, and a shaft 98 extending from the distal end 96 of the handle 92. As best shown in **Figs. 9** and **10**, the handle 92 has a lumen 100 in which a portion of the shaft 98 is permanently mounted in using suitable means, such as bonding. The handle 92 and shaft 98 may be composed of suitable rigid materials, such as stainless steel or polyethylene. As will be described in further detail below, the proximal end 94 of the handle 92 will serve as a lead-in when the lead body 16 to which the respective temporary identification device 70 is mounted is introduced through the tunneling straw 68.

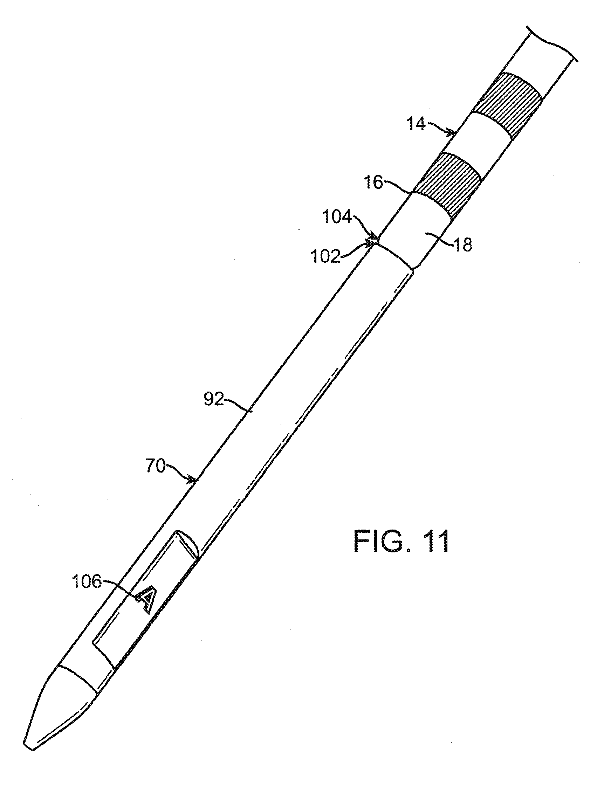

The shaft 98 of each temporary identification device 70 is sized to be firmly and removably received (e.g., by press fitting) within the respective lumen 30 extending with the lead body 16 of each stimulation lead 14, as shown in **Fig. 11**. For the purposes of this specification, a shaft is firmly received within a lumen if, when inserted into the lumen, the frictional force created between the shaft and lumen is equal to or greater than the maximum force created by the gravitational weight of the shaft. To this end, the diameter of the shaft 98 is preferably at least equal to the diameter of the lumen 30, and had a length (e.g., in the range of 1 cm to 8 cm) to create the frictional force necessary for it to be firmly received within the lumen 30. Alternatively, a slight bow or one or more bend(s) can be placed in a shaft 98 that has a smaller diameter shaft to create the interference fit between the shaft 98 and the lumen 30.

The cross-sectional of the handle 92 of each temporary identification device 70 is sized such that its distal end 96 abuts the proximal end 18 of the respective lead body 16 (shown in **Fig. 11**) when the respective shaft 98 is fully received within the lumen 30 of the lead body 16. In particular, each handle 92 has a distal-facing surface 102 that abuts a proximal facing surface 104 of the respective lead body 16. Preferably, the cross-sectional shape and cross-sectional size of the distal end 96 of each handle 92 matches the cross-sectional shape and cross-sectional size of the proximal end 18 of each lead body 16, such that when the shaft 98 is fully inserted into the lumen 30, the exterior surfaces of the handle 92 and respective lead body 16 act as a contiguous surface. In the illustrated embodiment, the proximal end 94 of each handle 92 tapers downward in the proximal direction, thereby allowing the handle 92 to more easily serve as a lead-in, as will be described in further detail below. The length of the handle 92 should be long enough for a physician to grasp with his or her fingers, but short enough to allow it to be easily introduced through the tunneling straw 68 when slightly bent. For example, the length of the handle 92 may be in the range of 1cm-3cm.

As best shown in **Fig. 6****,** each of the handles 92 has a different identifier 106, so that the handles 92, and thus, the lead bodies 16 to which they are mounted, can be differentiated from each other. In the illustrated embodiment, each identifier 106 takes the form of a different alpha-numerical character molded onto the handle 92. Alternatively, the alpha-numerical characters can be painted or printed on the handle 92. Although letters from the Roman alphabet (in this case, letters "A," "B," "C," and "D") are shown as the identifiers 106, letters from other alphabets (e.g., Hebrew, Arabic, Greek, Russian, Sanskrit, etc.) can be used. Also, numerals, such as cardinal numbers (e.g., "one," "two", "three," and "four," or Arabic symbols "1," "2," "3," and "4" or Roman symbols "I," "II," "III," and "IV") or ordinal numbers (e.g., the names "first," "second," "third," and "fourth," or the symbols "1^{st}," "2^{nd}," "3^{rd}," and "4^{th}") can be used. The identifiers 106 may also take the form of different colors (e.g., green, blue, pink, yellow, etc.) that can be printed, painted, or molded onto the handle 92) or different geometric shapes (e.g., triangles, rectangles, circles, trapezoids, etc.) can be printed or molded into the handle 92, for example, as circumferential bands.

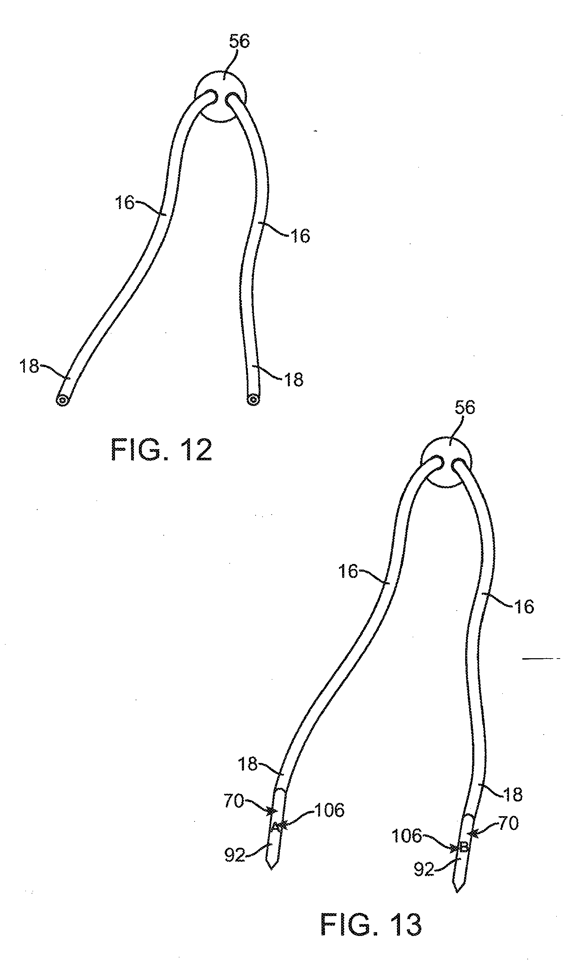

Referring now to **Figs. 12-16****,** as well as **Figs. 4-5****,** a method of implanting the stimulation leads 14 within a patient will now be described. First, the stimulation leads 14 are percutaneously implanted within the epidural space 52 (shown in **Fig. 4**) of the patient, such that the proximal ends 18 of the lead bodies 16 extend out from the exit point 56 of the patient, as shown in **Fig. 12****.** This is accomplished in a conventional manner under fluoroscopy using the needle 62 and the stylet 64.

For example, the needle 62 with an obturator (not shown) can be inserted through the back into the epidural space 52 of the patient. The obturator is then removed from the needle 62 to open the lumen 74, and a syringe (not shown) is inserted in the needle 62 to inject saline (3-5 cc) to ensure the needle tip has entered the epidural space 52. The stylet 64 is then inserted into the central lumen 30 of the stimulation leads 14 through the respective proximal end 18 of the lead body 16 to provide the stimulation lead 14 with the necessary rigidity, and the stimulation lead 14 with the stylet 64 is passed through the needle 62 into the epidural space 52. The other stimulation lead 14 can be introduced into the epidural space 52 in the same manner. After the stimulation leads 14 are placed, the needle 62 is then pulled out, and an anchor (not shown) is placed around the stimulation leads 14 at the exit point 56 and sutured in place to prevent movement of the stimulation leads 14.

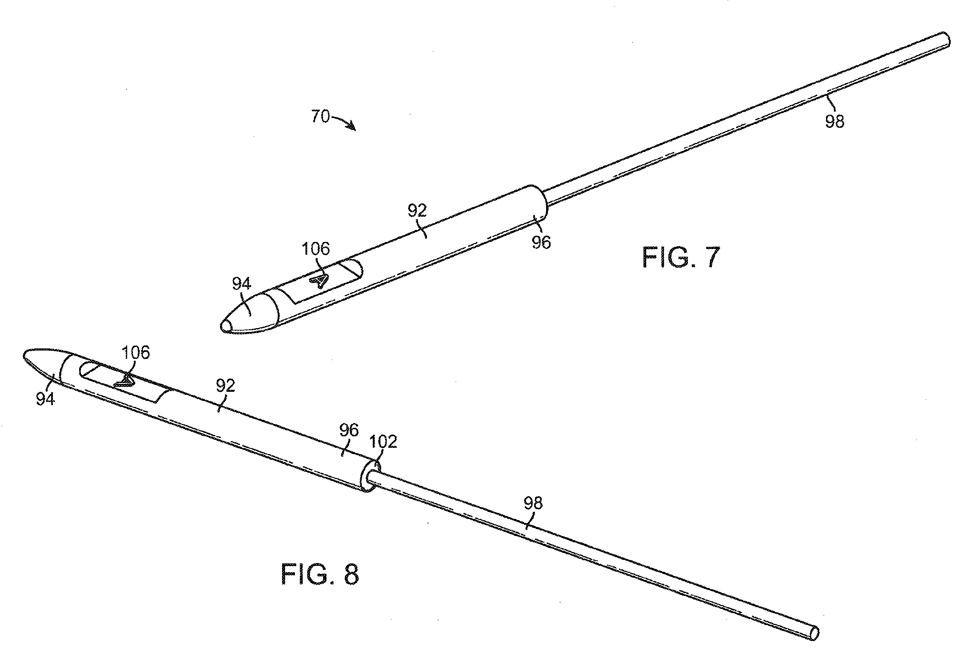

Significantly, the temporary identification devices 70 can be used to ensure that the lead bodies 16 (or lead bodies 36) are able to be easily identified through the remainder of the implantation process. In particular, a temporary identification device 70 can be mounted to each lead body 16 prior to or immediately after the respective stimulation lead 14 is introduced into the patient, so that the temporary identification device 70 can eventually be correlated to the location of the electrodes 22 that are associated with the lead body 16 to which the temporary identification device 70 is mounted, as illustrated in **Fig. 13****.** This is accomplished by first removing the stylet 64 from the lumen 30 of the lead body 16, and then inserting the shaft 98 of the respective temporary identification device 70 into the lumen 30 until the distal-facing surface 102 of the handle 92 abuts the proximal-facing surface 104 of the lead body 16 (shown in **Figs. 8** and **11**).

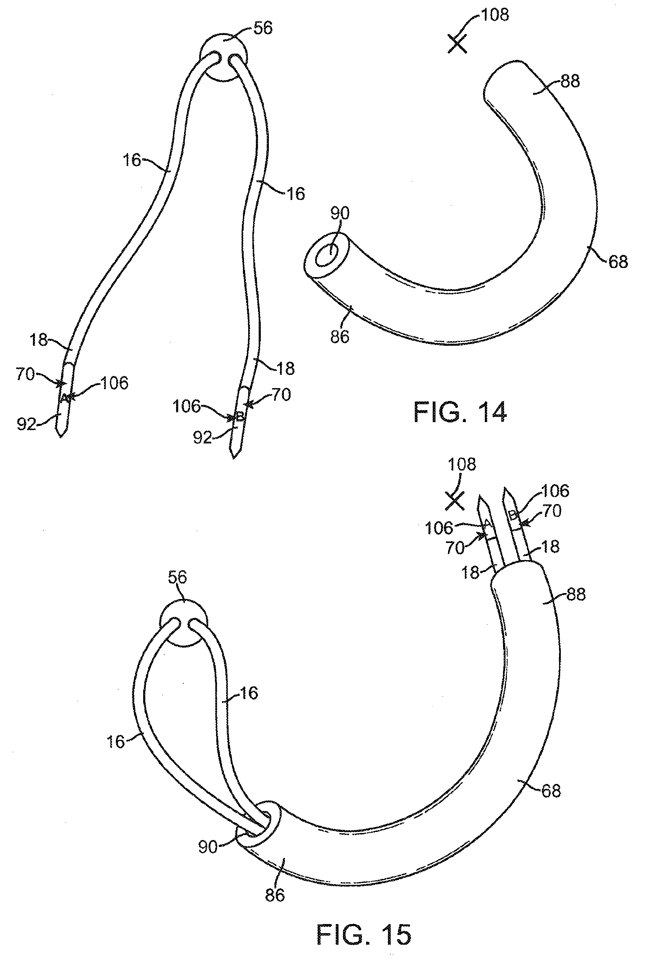

After the temporary identification devices 70 are mounted to the respective lead bodies 16, a tunnel is subcutaneously created from the exit point 56 on the back of the patient to the implantation site of the neurostimulator 12. This can be accomplished in a conventional manner using the tunneling tool 66 by advancing the distal end 80 of the shaft 76, with the tunneling straw 68 retained on the shaft 76 between the blunt tip 80 and the handle 92, underneath the patient's skin to create the tunnel from the lead exit point 56 to an implantation site 108 (shown in **Fig. 14**) of the neurostimulator 12. While the shaft 98 is in the tunnel, the handle 92 can then be removed from the shaft 98 (e.g., by unscrewing the handle 82) at the proximal end of the tunnel, and the shaft 98 with the blunt distal tip 80 removed at the distal end of the tunnel, thereby leaving in place the tunneling straw 68, which subcutaneously extends from the lead exit point 56 to the implantation site 108, as illustrated in **Fig. 14****.**

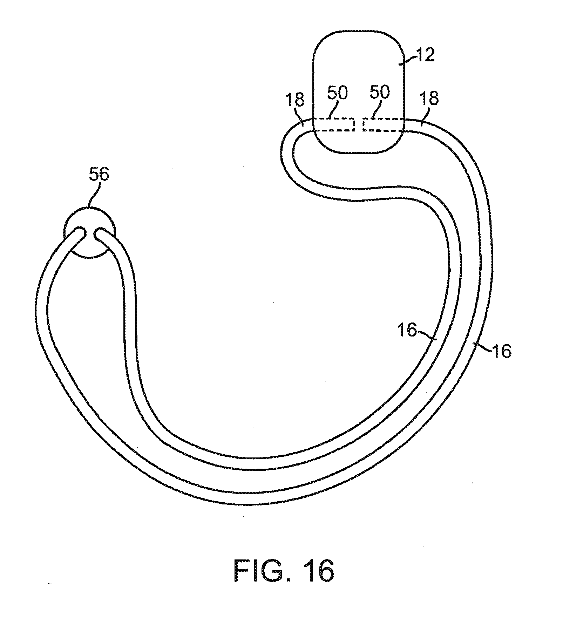

Next, the proximal ends 18 of the lead bodies 16, with the proximal ends 94 of the respective temporary identification devices 70 used as lead-ins, are advanced into the proximal end 86 of the tunneling straw 68, through the lumen 90, and out of the distal end 88 of the tunneling straw 68, as shown in **Fig. 15****.** The neurostimulator 12 is then implanted at the implantation site 108, and the lead bodies 16 are identified by examining the identifiers 106 of the temporary identification devices 70 extending from the distal end 88 of the tunneling straw 68. The tunneling straw 68 is removed over the proximal ends 18 of the lead bodies 16, the temporary identification devices 70 are then removed from the respective lumens 30 of the lead bodies 16, and the proximal ends 18 of the lead bodies 16 are then inserted into the respective connectors 50 (shown in phantom) of the implanted neurostimulator 12, as illustrated in **Fig. 16****.**

Preferably, the identification devices 70 are removed from the respective lead bodies 16 and the proximal ends 18 of the lead bodies 16 inserted into the connectors 50 of the implanted neurostimulator 12 one at a time (i.e., the identification device 70 on one of the lead bodies 16 is removed, the proximal end 18 of that lead body 16 inserted into a connector 50 of the neurostimulator 12, the identification device 70 on the other of the lead bodies 16 is removed, and then the proximal 18 of that lead body 16 inserted into the other connector 50 of the neurostimulator 12), so as not to confuse the identification of the lead bodies 16. If extension leads are used, the proximal ends 18 of the lead bodies 16 will be inserted into the distal ends of the extension leads, and then the proximal ends of the extension leads will be inserted into the respective connectors 50 of the neurostimulator 12.

In the alternative case where the surgical stimulation lead 34 (illustrated in **Fig. 3**) is to be implanted within the patient, the stimulation lead can be introduced into the epidural space 52 of the patient via a surgical opening formed using a conventional procedure, such as a laminectomy, and the procedure described with respect to **Figs. 13-16** can be used to identify the proximal ends 38 of the lead bodies 36 of the stimulation lead 34 and subcutaneously route the lead bodies 36 to the neurostimulator 12.

## Claims

1. An implantable lead kit (60) comprising:
an electrical lead (14, 34) including an elongated lead body (16, 36) having a proximal end (18, 38), at least one distally-located electrode (22), and a lumen (30) disposed within the proximal end (18, 38) of the lead body (16, 36); and
a plurality of temporary identification devices (70), each of which includes a handle (92) having a different identifier (106) and a shaft (98) extending from the handle (92), each shaft sized to be firmly and removably received within the lumen (30) of the electrical lead.

2. The implantable lead kit (60) of claim 1, wherein the electrical lead (64) is a percutaneous lead (14).

3. The implantable lead kit (60) of claim 2, further comprising another percutaneous lead (14) including an elongated lead body (16) having a proximal end (18), at least one distally-located electrode (22), and a lumen (30) disposed within the proximal end of the lead body, wherein each shaft (98) is sized to be firmly and removably received within the lumen (30) of the other percutaneous lead.

4. The implantable lead kit (60) of claim 1, wherein the electrical lead (34) is a surgical lead (34) that includes a paddle-shaped membrane (35) from which the lead body (36) extends, and each of the at least one electrode (22) is mounted on one side of the paddle-shaped membrane (35).

5. The implantable lead kit (60) of claim 4, wherein the surgical lead (34) includes another elongated lead body (36) having a proximal end (38), and another lumen (30) disposed within the proximal end (38) of the other lead body (36), and wherein each shaft (98) is sized to be firmly and removably received within the other lumen (30) of the surgical lead.

6. The implantable lead kit (60) of claim 1, wherein the electrical lead (14, 34) further includes at least one terminal carried by the proximal end (18, 38) of the lead body (16, 36) and at least one electrical conductor respectively connected between the at least one electrode (22) and the at least one terminal.

7. The implantable lead kit (60) of claim 1, wherein each handle (92) has a distal-facing surface (102) sized to abut a proximal-facing surface (104) of the lead body (16, 36) when the respective shaft (98) is fully received within the lumen of the electrical lead.

8. The implantable lead kit (60) of claim 1, wherein each handle (92) has a distal end (96) having substantially the same cross-sectional size as a cross-sectional size of the proximal end (18, 38) of the lead body (16, 36).

9. The implantable lead kit (60) of claim 1, wherein each handle (92) is tapered downward in the proximal direction.

10. The implantable lead kit (60) of claim 1, wherein the different identifier (106) is a different alpha-numeric character.

11. The implantable lead kit (60) of claim 1, wherein the different identifier (106) is a color.

12. The implantable lead kit (60) of claim 1, wherein the different identifier (106) is a geometric shape.

13. The implantable lead kit (60) of claim 1, further comprising a stylet (64) sized to be removably received within the lumen (30) of the electrical lead (14, 34).

14. The implantable lead kit (60) of claim 1, further comprising:
a tunneling tool (66) configured for subcutaneously creating a tunnel within a patient; and
a tunneling straw (68) configured for being introduced within the tunnel and further configured for receiving the proximal end (18, 38) of the lead body (16, 36).

## Patentansprüche

1. Implantierbarer Leitungssatz (60), der aufweist:
eine elektrische Leitung (14, 34) mit einem länglichen Leitungskörper (16, 36), der ein proximales Ende (18, 38) hat, mindestens einer distal liegenden Elektrode (22) und einem im proximalen Ende (18, 38) des Leitungskörpers (16, 36) angeordneten Lumen (30); und
mehrere temporäre Identifizierungsvorrichtungen (70), von denen jede einen Griff (92) mit einem unterschiedlichen Bezeichner (106) und einen sich vom Griff (92) erstreckenden Schaft (98) aufweist, wobei jeder Schaft so bemessen ist, dass er im Lumen (30) der elektrischen Leitung fest und entfernbar aufgenommen ist.

2. Implantierbarer Leitungssatz (60) nach Anspruch 1, wobei die elektrische Leitung (14) eine perkutane Leitung (14) ist.

3. Implantierbarer Leitungssatz (60) nach Anspruch 2, der ferner aufweist: eine weitere perkutane Leitung (14) mit einem länglichen Leitungskörper (16), der ein proximales Ende (18) hat, mindestens einer distal liegenden Elektrode (22) und einem im proximalen Ende des Leitungskörpers angeordneten Lumen (30), wobei jeder Schaft (98) so bemessen ist, dass er im Lumen (30) der weiteren perkutanen Leitung fest und entfernbar aufgenommen ist.

4. Implantierbarer Leitungssatz (60) nach Anspruch 1, wobei die elektrische Leitung (34) eine chirurgische Leitung (34) ist, die eine paddelförmige Membran (35) aufweist, von der sich der Leitungskörper (36) erstreckt, und jede der mindestens einen Elektrode (22) auf einer Seite der paddelförmigen Membran (35) angeordnet ist.

5. Implantierbarer Leitungssatz (60) nach Anspruch 4, wobei die chirurgische Leitung (34) einen weiteren länglichen Leitungskörper (36) mit einem proximalen Ende (38) und einem weiteren Lumen (30) aufweist, das im proximalen Ende (38) des weiteren Leitungskörpers (36) angeordnet ist, und wobei jeder Schaft (98) so bemessen ist, dass er im weiteren Lumen (30) der chirurgischen Leitung fest und entfernbar aufgenommen ist.

6. Implantierbarer Leitungssatz (60) nach Anspruch 1, wobei die elektrische Leitung (14, 34) ferner mindestens einen Anschluss, der vom proximalen Ende (18, 38) des Leitwigskörpers (16, 36) mitgeführt wird, und mindestens einen elektrischen Leiter aufweist, der jeweils zwischen der mindestens einen Elektrode (22) und dem mindestens einen Anschluss verbunden ist.

7. Implantierbarer Leitungssatz (60) nach Anspruch 1, wobei jeder Griff (92) eine distal weisende Oberfläche (102) hat, die so bemessen ist, dass sie an eine proximal weisende Oberfläche (104) des Leitungskörpers (16, 36) anstößt, wenn der jeweilige Schaft (98) im Lumen der elektrischen Leitung voll aufgenommen ist.

8. Implantierbarer Leitungssatz (60) nach Anspruch 1, wobei jeder Griff (92) ein distales Ende (96) mit im Wesentlichen der gleichen Querschnittgröße wie eine Querschnittgröße des proximalen Endes (18, 3 8) des Leitungskörpers (16, 3 6) hat.

9. Implantierbarer Leitungssatz (60) nach Anspruch 1, wobei jeder Griff (92) in proximaler Richtung nach unten zuläuft.

10. Implantierbarer Leitungssatz (60) nach Anspruch 1, wobei der unterschiedliche Bezeichner (106) ein unterschiedliches alphanumerisches Zeichen ist.

11. Implantierbarer Leitungssatz (60) nach Anspruch 1, wobei der unterschiedliche Bezeichner (106) eine Farbe ist.

12. Implantierbarer Leitungssatz (60) nach Anspruch 1, wobei der unterschiedliche Bezeichner (106) eine geometrische Form ist.

13. Implantierbarer Leitungssatz (60) nach Anspruch 1, der ferner ein Stilett (64) aufweist, das so bemessen ist, dass es im Lumen (30) der elektrischen Leitung (14, 34) entfernbar aufgenommen ist.

14. Implantierbarer Leitungssatz (60) nach Anspruch 1, der ferner aufweist:
ein Durchtunnelungswerkzeug (66), das zum subkutanen Erzeugen eines Tunnels in einem Patient konfiguriert ist; und
einen Durchtunnelungshalm (68), der zum Einführen im Tunnel konfiguriert und ferner zum Aufnehmen des proximalen Endes (18, 38) des Leitungskörpers (16, 36) konfiguriert ist.

## Revendications

1. Kit de dérivation implantable (60) comprenant:
une dérivation électrique (14, 34) englobant un corps de dérivation allongé (16, 36) possédant une extrémité proximale (18, 38), au moins une électrode (22) disposée en position distale et une lumière (30) disposée au sein de l'extrémité proximale (18, 38) du corps de dérivation (16, 36) ; et
plusieurs dispositifs d'identification temporaire (70), chacun englobant une poignée (92) possédant un identificateur différent (106) et une gaine (98) s'étendant à partir de la poignée (92), chaque gaine étant dimensionnée pour venir se loger fermement et de manière amovible au sein de la lumière (30) de la dérivation électrique.

2. Kit de dérivation implantable (60) selon la revendication 1, dans lequel la dérivation électrique (14) est une dérivation percutanée (14).

3. Kit de dérivation implantable (60) selon la revendication 2, comprenant en outre une autre dérivation percutanée (14) englobant un corps de dérivation allongé (16) possédant une extrémité proximale (18), au moins une électrode (22) disposée en position distale et une lumière (30) disposée au sein de l'extrémité proximale du corps de dérivation, chaque gaine (98) étant dimensionnée pour venir se loger fermement et de manière amovible au sein de la lumière (30) de l'autre dérivation percutanée.

4. Kit de dérivation implantable (60) selon la revendication 1, dans lequel la dérivation électrique (34) est une dérivation chirurgicale (34) qui englobe une membrane (35) en forme de palette à partir de laquelle s'étend le corps de dérivation (36), et chacune desdites au moins une électrode (22) est montée sur un côté de la membrane (35) en forme de palette.

5. Kit de dérivation implantable (60) selon la revendication 4, dans lequel la dérivation chirurgicale (34) englobe un autre corps de dérivation allongé (36) possédant une extrémité proximale (38), et une autre lumière (30) disposée au sein de l'extrémité proximale (38) de l'autre corps de dérivation (36), et dans lequel chaque gaine (98) est dimensionnée pour venir se loger fermement et de manière amovible au sein de l'autre lumière (30) de la dérivation chirurgicale.

6. Kit de dérivation implantable (60) selon la revendication 1, dans lequel la dérivation électrique (14, 34) englobe en outre au moins une borne supportée par l'extrémité proximale (18, 38) du corps de dérivation (16, 36) et au moins un conducteur électrique respectivement relié entre ladite au moins une électrode (22) et ladite au moins une borne.

7. Kit de dérivation implantable (60) selon la revendication 1, dans lequel chaque poignée (92) possède une surface (102) orientée en direction distale, dimensionnée pour venir buter contre une surface (104) du corps de dérivation (16, 36), orientée en direction proximale, lorsque la gaine respective (98) est complètement insérée au sein de la lumière de la dérivation électrique.

8. Kit de dérivation implantable (60) selon la revendication 1, dans lequel chaque poignée (92) possède une extrémité distale (96) possédant essentiellement la même dimension en section transversale que la dimension en section transversale de l'extrémité proximale (18, 38) du corps de dérivation (16, 36).

9. Kit de dérivation implantable (60) selon la revendication 1, dans lequel chaque poignée (92) présente une conicité descendante dans la direction proximale.

10. Kit de dérivation implantable (60) selon la revendication 1, dans lequel l'identificateur différent (106) est un caractère alphanumérique différent.

11. Kit de dérivation implantable (60) selon la revendication 1, dans lequel l'identificateur différent (106) est une couleur.

12. Kit de dérivation implantable (60) selon la revendication 1, dans lequel l'identificateur différent (106) est une forme géométrique.

13. Kit de dérivation implantable (60) selon la revendication 1, comprenant en outre un stylet (64) dimensionné pour venir se loger de manière amovible au sein de la lumière (30) de la dérivation électrique (14, 34).

14. Kit de dérivation implantable (60) selon la revendication 1, comprenant en outre :
un ustensile de tunnellisation (66) configuré pour créer, par voie sous-cutanée, un tunnel au sein d'un patient ; et
une paillette de tunnellisation (68) configurée pour s'introduire au sein du tunnel et configurée en outre pour que vienne s'y loger l'extrémité proximale (18, 38) du corps de dérivation (16, 36).
